# EUROPEAN PATENT APPLICATION

(11) **EP 2 860 238 A1**
(43) Date of publication of application: **15.04.2015**
(21) Application number: 14188519.4
(22) Date of filing: 10.10.2014
(51) Int. Cl.: C12M 1/00, C12N 1/20

(54) **Culture bag**

(30) Priority: 10.10.2013 IT MO20130284
(71) Applicant: GENERON S.r.l., 41126 Santa Maria di Mugnano (MO) (IT)
(72) Inventor: Ferrari, Giuseppe, 41126 MODENA - S. Maria di Mugnano (IT)
(74) Representative: Brunacci, Marco

(57) **Abstract**

The product (1) for microbiology usable in bead beater homogenizers or similar devices comprises a bag (2) which can be positioned inside a bead beater homogenizer (O), and a substance or a mix of substances (3) for microbiology, housed inside the bag (2), dehydrated and present in a predefined quantity needed for that specific use.

## Description

The present invention relates to a product for microbiology usable in bead beater homogenizers or the like, usable in particular to perform analytical microbiology procedures on foods, animal and vegetable tissues, pharmacological or formulated products, including veterinary products.

It is known that dehydrated microbiology culture mediums, intended as a mix of substances, are today available on the market in packs of many sizes.

The most common type of pack envisages the use of powdered dehydrate packed in cans of different sizes from 100 and 500 grams, right up to various kilogram weights, according to the particular type of use.

A further type of microbiology culture medium pack envisages the use of the culture medium in liquid state, dissolved by adding sterile water or other solvent.

The product in this physical rehydrated state is available on the market in test tubes, bottles and bags of various sizes.

The sizes as described above, in use to date, do however have a number of drawbacks for the user.

In particular, in the case of use of dehydrated powdered products packed in cans or in other similar packs, each can contains the dehydrated product in greater weight compared to the single dose required.

Consequently, the user must necessarily take out the necessary quantity of product according to the test to be performed, weigh it, hydrate it with water, sterilize it, dispense it in a special bag for the bead beater homogenizer and also add the sample to be tested.

All this inevitably takes up a lot of the user's time, besides representing a risk of contamination due to the numerous product handling phases.

With regard, instead, to the case of the use of an already rehydrated microbiology culture medium, which is therefore ready for use, the user must in any case address the far from negligible problem of moving a bag or a container full of liquids, a bag which could even be very heavy.

In particular, the weight and/or the fragility of such products represent a major logistics problem in terms of costs and shipment, besides the serious risk of receiving products broken during the normal supply phases.

Furthermore, the bag used can be subject to the dispersion of liquid due to breakage in the microbiology laboratory, a place typically full of sterile and fragile equipment.

A further problem that cannot be ignored is the life span of the product which, once rehydrated, is considerably reduced compared to the corresponding dehydrated product.

The user therefore finds himself/herself forced to quickly use the entire contents of the bag and must necessarily manage warehouse turnovers to prevent the ready products from expiring.

The main aim of the present invention is to provide a product for microbiology usable in bead beater homogenizers or the like which allows an easier and quicker use compared to the solutions of known type.

Another object of the previous invention is to provide a product for microbiology usable in bead beater homogenizers or the like which allows to minimize the risk of contamination.

Another object of the present invention is to provide a product for microbiology usable in bead beater homogenizers or the like which allows to minimize the risk of damage.

Another object of the present invention is to provide a product for microbiology usable in bead beater homogenizers or the like which has a longer life span than the solutions of known type with the hydrated product.

Another object of the present invention is to provide a product for microbiology usable in bead beater homogenizers or the like, which allows to overcome the mentioned drawbacks of the prior art within the ambit of a simple, rational and affordable solution.

The above mentioned objects are achieved by the present product for microbiology usable in bead beater homogenizers or the like, according to claim 1.

Other characteristics and advantages of the present invention will become better evident from the description of a preferred but not exclusive embodiment of a product for microbiology usable in bead beater homogenizers or the like, illustrated by way of an indicative, but not limitative, example in the accompanying drawings in which:
Figures 1, 2, 3 and 4 illustrate respective embodiments of the product according to the invention;
Figures 5 and 6 illustrate the use of the product according to the invention.

With particular reference to these figures, globally indicated by reference numeral 1 is a product for microbiology usable in bead beater homogenizers of conventional type or in similar devices.

In particular, the product 1 is a ready product usable to carry out analytical microbiology procedures on foods, animal and vegetable tissues, pharmacological or formulated products, including veterinary products.

The product 1 comprises a bag 2 positionable inside a bead beater homogenizer O of conventional type.

The bag 2 has a substantially rectangular shape and has dimensions varying between 75 mm and 295 mm in width and between 90 mm and 510 mm in height.

In particular, the bag 2 is compatible with the typical dimensions for bead beater homogenizers O suitable for containing a minimum of 40 ml up to a maximum of 1000 ml of overall capacity.

Preferably, the bag 2 has dimensions equal to 180 mm in width and 310 mm in height, with a capacity of 400 ml.

Preferably, the bag 2 is made of opaque or transparent food-grade polyethylene. Preferably, the material of which the bag 2 is made is a single-layer or multilayer simple polymer of Polyethylene (PE), Polypropylene (PP), Polyvinyl chloride (PVC), Polyethylene terephthalate (PET) or other synthetic and natural polymers.

If necessary, the bag 2 can be fitted with a strip or total filtering accessory. Advantageously, inside the bag 2, the product 1 comprises a substance or a mix of substances 3 for microbiology, dehydrated and present in the exact and predefined quantity needed for that specific use.

In particular, such substance or mix of substances 3 is able to form a mix typically called for microbiology culture medium.

The substance or mix of substances 3 is present in a quantity that varies from 1 gram to 50 grams in total, depending on the specific use it is intended for.

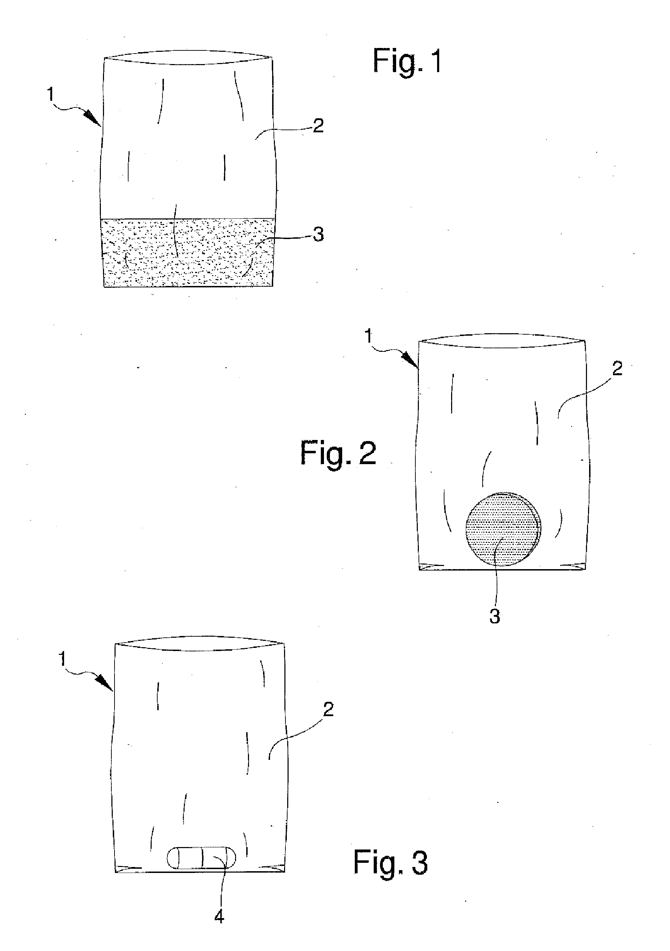

In particular, with reference to a first embodiment of the product 1 shown in figure 1, the substance or mix of substances 3 can be distributed in loose state and in the form of dehydrated powder or granular dehydrated powder directly inside the bag 2.

With reference to a second embodiment of the product 1, shown in figure 2, the substance or mix of substances 3 can be distributed in the form of one or more tablets housed inside the bag 2.

With reference to a third embodiment of the product 1, shown in figure 3, the substance or mix of substances 3 can be distributed in the form of dehydrated powder or granular dehydrated powder and inside one or more capsules 4 soluble in water or in other suitable solvent and housed in the bag 2.

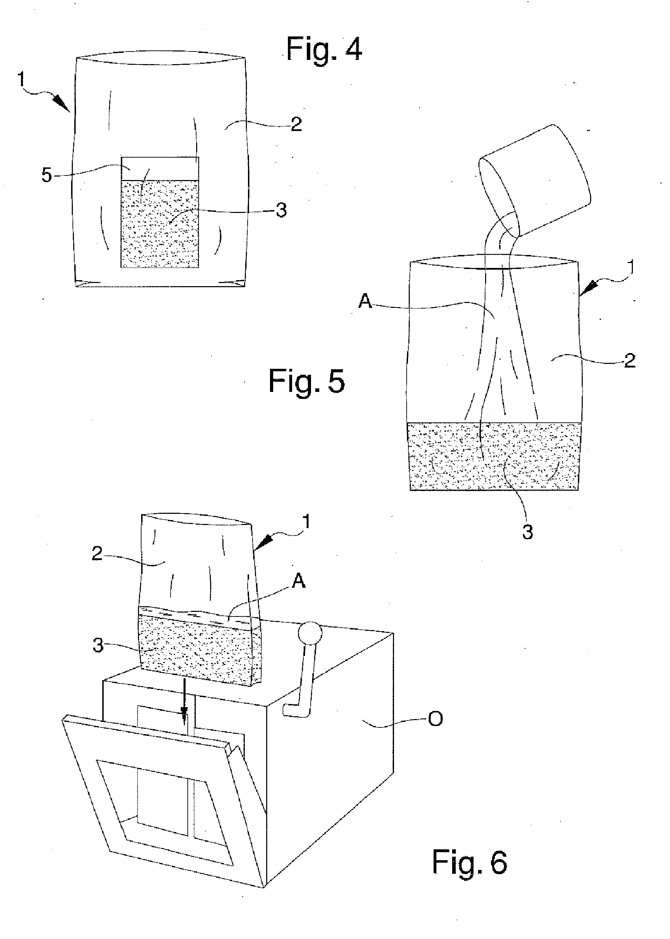

With reference to a fourth embodiment of the product 1, shown in figure 4, the substance or mix of substances 3 can be distributed in the form of dehydrated powder or granular dehydrated powder and inside one or more water-soluble bags 5, soluble in water or in another suitable solvent, and housed in the bag 2. Usefully, the substance or the mix of substances 3 is sealed and sterilized inside the bag 2 and is ready for use.

Preferably, but with no limitation, the mix of substances 3 is selected from the group comprising the following mixes.

"Buffered peptone water (BPW)" mix formed by (ratio expressed in grams per litre):
- from 5 to 15 g of Peptone;
- from 2 to 8 g of Sodium Chloride;
- from 5 to 15 g of Sodium Phosphate Dibasic dodecahydrate or, alternatively, from 2 to 8 g of Sodium Phosphate Dibasic anhydrous;
- from 0.5 to 4 g of Potassium Phosphate Monobasic anhydrous.

Preferably, this mix is formed by:
- 10 g of Peptone;
- 5 g of Sodium Chloride;
- 9 g of Sodium Phosphate Dibasic dodecahydrate or, alternatively, 3.5 g of Sodium Phosphate Dibasic anhydrous;
- 1.5 g of Potassium Phosphate Monobasic anhydrous.

Moreover, the use of further similar formulations that are functionally suitable for the purpose is not to be ruled out.

"Peptone Saline Diluent" mix formed by (ratio expressed in grams per litre):
- from 1 to 20 g of Peptone;
- from 1 to 20 g of Sodium Chloride.

Preferably, this mix is formed by:
- 1 g of Peptone;
- 8.5 g of Sodium Chloride.

Moreover, the use of further similar formulations that are functionally suitable for the purpose is not to be ruled out.

"Peptone Water (BPW)" mix formed by (ratio expressed in grams per litre):
- from 1 to 20 g of Peptone;
- from 1 to 20 g of Sodium Chloride.

Preferably, this mix is formed by:
- 10 g of Peptone;
- 5 g of Sodium Chloride.

Moreover, the use of further similar formulations that are functionally suitable for the purpose is not however to be ruled out.

"Half Fraser" mix formed by (ratio expressed in grams per litre):
- from 2 to 8 g of Peptone;
- from 2 to 8 g of Tryptone;
- from 2 to 8 g of Meat extract;
- from 2 to 8 g of Yeast Extract;
- from 10 to 30 g of Sodium Chloride;
- from 10 to 30 g of Sodium Phosphate Dibasic dodecahydrate or, alternatively, from 5 to 15 g of Sodium Phosphate Dibasic anhydrous;
- from 1 to 5 g of Potassium Phosphate Monobasic;
- from 0.5 to 4 g of Esculin;
- from 1 to 5 g of Lithium Chloride;
- from 0.3 to 2 g of Ferric Ammonium Citrate;
- from 0.01 to 1 g of Nalidixic Acid;
- from 0.01 to 1 g of Acriflavine Hydrochloride.

Preferably, this mix is formed by:
- 5 g of Peptone;
- 5 g of Tryptone;
- 5 g of Meat extract;
- 5 g of Yeast Extract;
- 20 g of Sodium Chloride;
- 12 g of Sodium Phosphate Dibasic dodecahydrate;
- 1.35 g of Potassium Phosphate Monobasic;
- 1 g of Esculin;
- 3 g of Lithium Chloride;
- 0.5 g of Ferric Ammonium Citrate;
- 0.0125 g of Acriflavine Hydrochloride;
- 0.01 g of Nalidixic Acid.

Moreover, the use of further similar formulations that are functionally suitable for the purpose is not however to be ruled out.

It is also not excluded the use of different mixes of substances 3.

The use of the product 1 is schematically illustrated in figures 5 and 6.

In particular, as shown in figure 5, the operator takes the product 1 as packed, opens the bag 2 and adds to the substance or mix of substances 3 water or a suitable solvent in a predefined quantity depending on the specific test to be performed.

Subsequently, as shown in figure 6, the bag 2 is positioned with the respective contents to be homogenized inside a conventional bead beater homogenizer O, and blocked inside this.

The homogenization procedure of the substance or the mix of substances 3 with the added solvent continues in accordance with prior art.

Consequently, the homogenization procedure is made considerably simpler compared to the current state of the art.

It has in practice been ascertained how the described invention achieves the proposed objects.

In particular, the fact is underlined that the product according to the invention solves problems of normal alternative supplies, inasmuch as it has a much longer life span than the respective products in liquid form (rehydrated), since it is in dehydrated form.

Furthermore, the product according to the invention reduces the risk of laboratory contamination compared to dehydrated products, inasmuch as it eliminates dosing operations for weighing by the operator.

The product according to the invention also eliminates customers' shipment problems with regard to the total weight, dimensions, logistics and handling, since it is without parts in glass or other fragile material or heavy liquids that would considerably increase shipment costs.

The product according to the invention reduces the risk of operator error, by limiting use operations.

Finally, the product according to the invention makes laboratory handling and use easier for users, being very light in weight, sterile and without water.

## Claims

1. Product (1) for microbiology usable in bead beater homogenizers or similar devices, **characterized by** the fact that it comprises a bag (2) which can be positioned inside a bead beater homogenizer (O), and a substance or a mix of substances (3) for microbiology, housed inside said bag (2), dehydrated and present in a predefined quantity needed for that specific use.

2. Product (1) according to claim 1, **characterized by** the fact that said substance or mix of substances (3) is in the form of loose dehydrated powder or loose granular dehydrated powder, distributed directly inside said bag (2).

3. Product (1) according to one or more of the preceding claims, **characterized by** the fact that said substance or mix of substances (3) is in the form of at least a tablet.

4. Product (1) according to one or more of the preceding claims, **characterized by** the fact that said substance or mix of substances (3) is in the form of dehydrated powder or granular dehydrated powder and is housed inside at least a capsule (4) soluble in water or in other suitable solvent.

5. Product (1) according to one or more of the preceding claims, **characterized by** the fact that said substance or mix of substances (3) is in the form of dehydrated powder or granular dehydrated powder and is housed inside at least a water-soluble bag (5) soluble in water or in other suitable solvent.

6. Product (1) according to one or more of the preceding claims, **characterized by** the fact that said substance or mix of substances (3) is sealed and sterilized inside said bag (2).

7. Product (1) according to one or more of the preceding claims, **characterized by** the fact that said bag (2) has a substantially rectangular shape and has dimensions between 75 mm and 295 mm in width and between 90 mm and 510 mm in height.

8. Product (1) according to one or more of the preceding claims, **characterized by** the fact that said bag (2) has a capacity that varies between a minimum of 40 ml up to a maximum of 1000 ml.

9. Product (1) according to one or more of the preceding claims, **characterized by** the fact that said bag (2) is made of opaque or transparent food-grade polyethylene or other polymers suitable for the purpose.

10. Product (1) according to one or more of the preceding claims, **characterized by** the fact that said substance or mix of substances (3) is present in a quantity variable from 1 gram to 50 grams in total, depending on the specific use it is intended for.

11. Product (1) according to one or more of the preceding claims, **characterized by** the fact that said substance or mix of substances (3) comprises, referred to grams per liter:
- from 5 g to 15 g of Peptone;
- from 2 g to 8 g of Sodium Chloride;
- from 5 g to 15 g of Sodium Phosphate Dibasic dodecahydrate or, alternatively, from 2 g to 8 g of Sodium Phosphate Dibasic anhydrous;
- from 0.5 g to 4 g of Potassium Phosphate Monobasic anhydrous.

12. Product (1) according to one or more of the preceding claims, **characterized by** the fact that said substance or mix of substances (3) comprises, referred to grams per liter:
- from 1 g to 20 g of Peptone;
- from 1 g to 20 g of Sodium Chloride.

13. Product (1) according to one or more of the preceding claims, **characterized by** the fact that said substance or mix of substances (3) comprises, referred to grams per liter:
- from 2 g to 8 g of Peptone;
- from 2 g to 8 g of Tryptone;
- from 2 g to 8 g of Meat extract;
- from 2 g to 8 g of Yeast extract;
- from 10 g to 30 g of Sodium Chloride;
- from 10 g to 30 g of Sodium Phosphate Dibasic dodecahydrate or, alternatively, from 5 g to 15 g of Sodium Phosphate Dibasic anhydrous;
- from 1 g to 5 g of Potassium Phosphate Monobasic;
- from 0.5 g to 4 g of Esculin;
- from 1 g to 5 g of Lithium Chloride;
- from 0.3 g to 2 g of Ferric Ammonium Citrate;
- from 0.01 g to 1 g of Nalidixic Acid;
- from 0.01 g to 1 g of Acriflavine Hydrochloride.
